# EUROPEAN PATENT SPECIFICATION

(11) **EP 4 025 164 B1**
(45) Date of publication and mention of the grant of the patent: **15.05.2024**
(21) Application number: 20768704.7
(22) Date of filing: 02.09.2020
(51) Int. Cl.: A61F 13/00, A61F 13/02

(54) **CONTOURED DRESSING FOR TISSUE THERAPY**
KONTURIERTER VERBAND ZUR GEWEBETHERAPIE
PANSEMENT PROFILÉ POUR LA THÉRAPIE TISSULAIRE

(30) Priority: 05.09.2019 US 201962896377 P
(43) Date of publication of application: 13.07.2022
(73) Proprietor: Solventum Intellectual Properties Company, Maplewood, MN 55144 (US)
(72) Inventor: REHBEIN, Jonathan G., San Antonio, Texas 78265 (US); PERKINS, Luke, San Antonio, Texas 78265 (US); RANDOLPH, Larry Tab, San Antonio, Texas 78265 (US); KAZALA, Richard Marvin, San Antonio, Texas 78265 (US)
(74) Representative: Simmons & Simmons
(86) International application number: PCT/IB2020/058168
(87) International publication number: WO 2021/044309

(56) References cited:
- US-A1- 2012 209 226
- US-A1- 2015 057 624
- US-A1- 2015 057 625
- US-A1- 2018 289 871

## Description

### TECHNICAL FIELD

This disclosure relates generally to medical treatment systems.

### BACKGROUND

Clinical studies and practice have shown that reducing pressure in proximity to a tissue site can augment and accelerate growth of new tissue at the tissue site. The applications of this phenomenon are numerous, but have proven particularly advantageous for treating wounds. Regardless of the etiology of a wound, whether trauma, surgery, or another cause, proper care of a wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," and "vacuum-assisted closure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

US 2015/0057624 A1 concerns a dressing interface with a moisture controlling feature and sealing function.

While the clinical benefits of negative-pressure therapy are widely known, the cost and complexity of negative-pressure therapy can be a limiting factor in its application, and the development and operation of negative-pressure systems, components, and processes continues to present significant challenges to manufacturers, healthcare providers, and patients.

### SUMMARY

Shortcomings with certain aspects of tissue treatment dressings, systems, and methods are addressed as shown and described in a variety of illustrative, non-limiting example embodiments herein.

The invention provides a system for treating a tissue site according to claim 1. Further optional features are provided in the dependent claims.

In some example embodiments, the contoured bolster may include or be formed of a porous foam, and may further include a first side, an opposing second side, and an edge between the first side and the second side. The sealing skin may cover at least a portion of the first side of the contoured bolster. The tissue housing may be positioned on the second side of the contoured bolster. The tissue housing may include a three-dimensional contact surface configured to receive at least a portion of the tissue site and to contact the tissue site in three-dimensions. The gasket member may be configured to create a sealed space between the sealing skin and the tissue site.

Also disclosed herein is a method for treating a tissue site that may include disposing a dressing assembly proximate to the tissue site. The dressing assembly may include a contoured bolster, a sealing skin, and a tissue housing. The contoured bolster may include a first side and an opposing second side. The sealing skin may be formed integrally with at least a portion of the first side of the dressing bolster, and the tissue housing may be formed on the second side of the contoured bolster. Further, the method may include inserting at least a portion of the tissue site into the tissue housing. Further, the method may include forming a sealed space between the sealing skin and the tissue site, and the contoured bolster may be positioned in the sealed space. Further, the method may include distributing reduced pressure to the tissue site through the contoured bolster.

Also disclosed herein is a method of manufacturing a dressing for treating a tissue site may include injecting an uncured foam material into a mold. Further, the method may include curing the foam material to form a contoured bolster according to a shape of the mold. The contoured bolster may include a first side, a second side opposite the first side, and an edge between the first side and the second side. Further, the method may include forming a sealing skin on at least a portion of the first side of the contoured bolster as the foam material cures, and positioning a gasket member proximate to the edge and on the second side of the contoured bolster.

Other features and advantages of the illustrative example embodiments will become apparent with reference to the drawings and detailed description that follow.

### BRIEF DESCRIPTION OF THE DRAWINGS

FIGURE 1 is a perspective view of an illustrative example embodiment of a system for treating a tissue site;
FIGURE 2 is a cross-section of a portion of an illustrative example embodiment of a dressing assembly depicted in FIGURE 1, taken along line 2-2;
FIGURE 3 is a perspective view of an illustrative example embodiment of a portion of a treatment system for treating a tissue site;
FIGURE 4 is a cross-section of an illustrative example embodiment of a dressing assembly depicted in FIGURE 3, taken along line 4-4;
FIGURE 5 is an exploded, perspective view of the example dressing assembly of FIGURE 4 in a state prior to assembly or deployment at a tissue site;
FIGURES 6A-6C are perspective views, with a portion shown in cross-section, of a portion of an illustrative example embodiment of a treatment system being deployed over a linear wound;
FIGURE 7 is a perspective view of another illustrative example embodiment of a dressing assembly according to this disclosure;
FIGURE 8A illustrates the dressing assembly of FIGURE 7 deployed at an illustrative tissue site;
FIGURE 8B illustrates another example embodiment of a dressing assembly deployed at the illustrative tissue site; and
FIGURE 9 is a flow chart illustrating a method of manufacturing an example dressing assembly according to this disclosure.

### DETAILED DESCRIPTION OF EXAMPLE EMBODIMENTS

The following description of example embodiments enables a person skilled in the art to make and use the subject matter set forth in the appended claims. Certain details already known in the art may be omitted. Further, the following detailed description is illustrative and non-limiting.

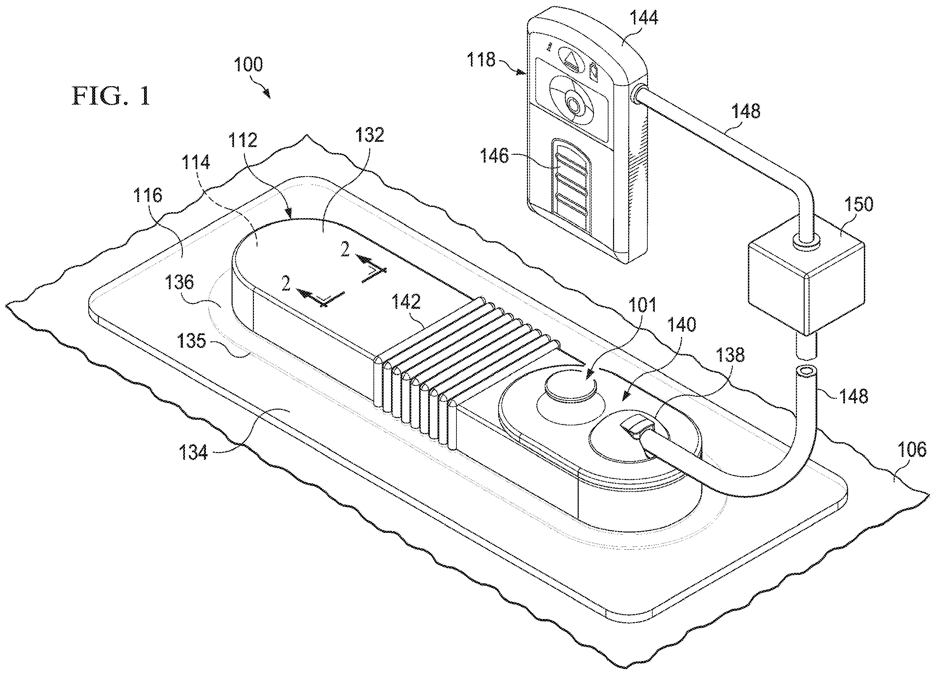

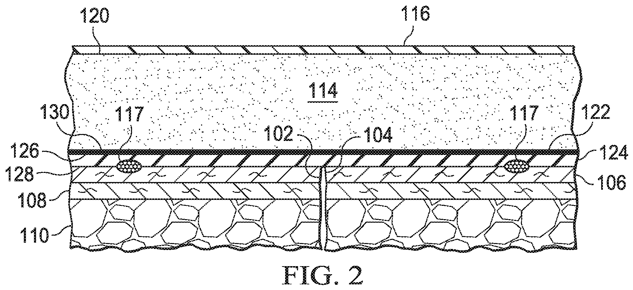

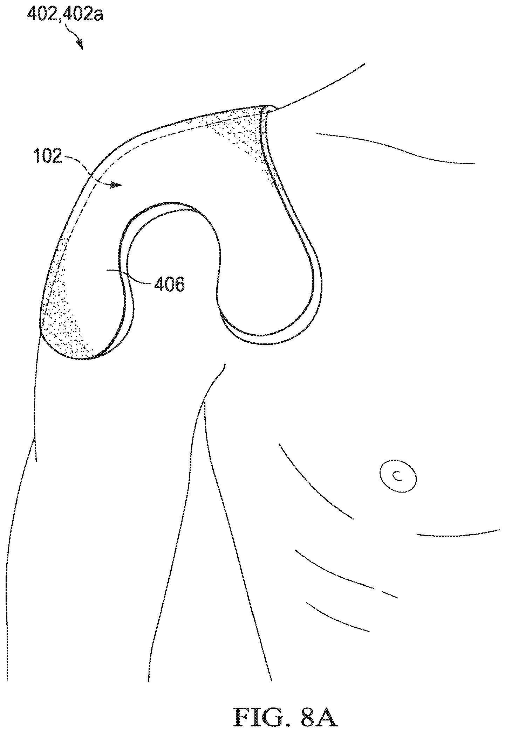

Referring primarily to FIGURES 1 and 2, presented is an illustrative, non-limiting example embodiment of a treatment system 100 for treating a tissue site 102. The tissue site 102 may be or may include, without limitation, an incision 104, wound, defect, or other tissue or feature. The incision 104 is shown extending through or involving epidermis 106, dermis 108, and subcutaneous tissue 110. The treatment system 100 may also be used with other tissue sites, and may be utilized with or without reduced pressure as described herein. Further, although the tissue site 102 is shown illustratively in FIGURE 2 as having a substantially flat or planar surface, in some examples, the tissue site 102 may include multi-dimensional or three-dimensional surfaces subject to treatment, such as, without limitation, a shoulder, knee, elbow, ankle, hand, breast, sacral region, or other multi-dimensional region of a patient. The tissue site 102 in the form of an example shoulder region is shown in FIGURES 8A-8B.

In some embodiments, the treatment system 100 may include a dressing assembly 112 having a dressing bolster 114, which may be referred to as a manifold member 114. In addition, some embodiments of the treatment system 100 may include a sealing member 116 and a reduced-pressure subsystem 118. Some embodiments of the treatment system 100 may also include a reduced-pressure indicator 101. While the treatment system 100 is shown in the context of a reduced-pressure dressing over an incision 104, the treatment system 100 may be used on other tissue sites, including open wounds. Further, features may be optionally added to or omitted from the treatment system 100, and various embodiments thereof, to suit different therapeutic applications, scenarios, or preferences, and thus, features described herein are not to be considered essential unless explicitly stated.

In some embodiments, the dressing bolster 114 may include a first side 120 and a second side 122 positioned opposite or facing opposite to the first side 120. The first side 120 of the dressing bolster 114 may be configured to face outward from or away from the tissue site 102, and the second side 122 of the dressing bolster 114 may be configured to face inward or toward the tissue site 102. The second side 122 of the dressing bolster 114 may also be referred to as a second, inward-facing side 122.

The dressing bolster 114 may be formed from any bolster material or manifold material that provides a vacuum space, or treatment space. Reduced pressure applied to the dressing bolster 114 may enhance the permeability of the dressing bolster 114. For example, the dressing bolster 114 may be formed from a porous and permeable foam or foam-like material, a member formed with pathways, a graft, a gauze, or any combination thereof. In some embodiments, the dressing bolster 114 may be a reticulated, open-cell polyurethane or polyether foam that may be fluid permeable. One example of a suitable foam material may be a GRANUFOAM^{™} material available from Kinetic Concepts, Inc. (KCI) of San Antonio, Texas.

The term "manifold" as used herein may refer to a substance or structure that may assist in applying reduced pressure to, delivering fluids to, or removing fluids from a tissue site. A manifold may include a plurality of flow channels or pathways. The plurality of flow channels may be interconnected to improve distribution of fluids provided to and removed from an area of tissue around the manifold. Examples of manifolds may include, without limitation, devices that have structural elements arranged to form flow channels, cellular foam, such as open-cell foam, porous tissue collections, and liquids, gels, and foams that include or cure to include flow channels.

The reticulated pores of the GRANUFOAM^{™} material may be helpful in carrying out the manifold function, but as stated above, other materials may be utilized. A material with a higher or lower density than the GRANUFOAM^{™} material may be desirable in some embodiments. This material may have, for example, a smaller pore size than the GRANUFOAM^{™} material. Among the many possible materials, the following may be used without limitation: GRANUFOAM^{™} material, FXI technical foam, gauze, a flexible channel-containing member, a graft, and other similar materials. In some embodiments, ionic silver may be added to the material, such as, for example, by a micro bonding process. Other substances, such as antimicrobial agents, may also be added to the material.

In some embodiments, a comfort layer 124 may be coupled, for example, by a heat bond 130 or other suitable technique to the second, inward-facing side 122 of the dressing bolster 114. The comfort layer 124 may include a first side 126 and a second side 128 positioned opposite or facing opposite to the first side 126. The first side 126 of the comfort layer 124 may be configured to face outward from or away from the tissue site 102, and the second side 128 of the comfort layer 124 may be configured to face inward or toward the tissue site 102. The first side 126 of the comfort layer 124 may be coupled to the second side 122 of the dressing bolster 114. Herein, the comfort layer 124 may also be referred to as an interfacial layer, a tissue interface layer, or tissue contact layer. Further, the second side 128 of the comfort layer 124 may also be referred to as a second, inward-facing side 128.

The comfort layer 124 may enhance patient comfort when the dressing bolster 114 is adjacent to or in contact with the epidermis 106 of a patient. The comfort layer 124 may be any material that helps prevent skin irritation and discomfort while allowing fluid transmission through the comfort layer 124. As non-limiting examples, the comfort layer 124 may include or be formed of a woven material, an elastic material, a polyester knit textile substrate, a non-woven material, or a fenestrated film. As another non-limiting example, an INTERDRY^{™} textile material available from Milliken Chemical, a division of Milliken & Company, Inc. of Spartanburg, South Carolina, may be utilized. In some embodiments, the comfort layer 124 may include antimicrobial substances, such as silver.

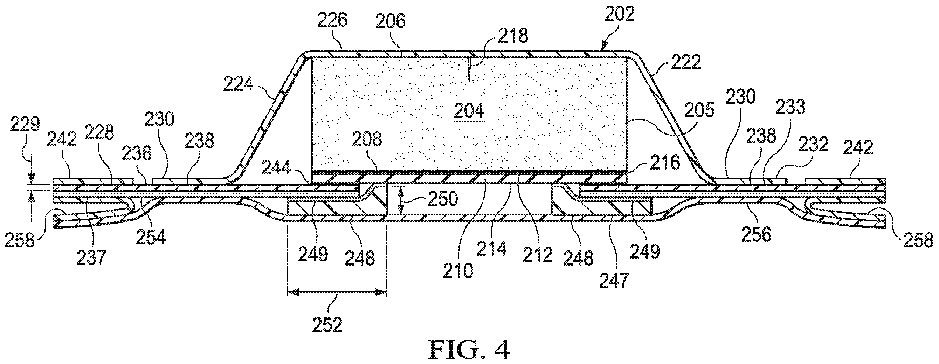

In some embodiments, the dressing bolster 114 may include a plurality of flexibility notches or recesses, analogous to notches 218 shown in FIGURE 4, for example, that may be lateral cuts in the dressing bolster 114 on the first side 120. The dressing bolster 114 may additionally or alternatively include one or more longitudinal cuts or notches, which may intersect the lateral cuts or notches. The flexibility notches may enhance the flexibility of the dressing bolster 114. The enhanced flexibility may be particularly useful when the dressing assembly 112 is applied over a joint or other area of movement on a patient. The flexibility notches may also take various shapes without limitation, such as, for example, hexagons, slits, or squares.

In some embodiments, the dressing bolster 114 may include lateral edges (not shown) that are orthogonal relative to the second, inward-facing side 122 of the dressing bolster 114. The lateral edges of the dressing bolster 114 may be analogous to lateral edges 205 of dressing bolster 204 depicted in FIGURE 4. The lateral edges of the dressing bolster 114 may also have a beveled edge or angled edge. The angled or beveled edge may help distribute shear stress between the dressing bolster 114 and the epidermis 106 of a patient. The lateral edges of the dressing bolster 114 may substantially correspond to lateral edges (not shown) of the comfort layer 124.

The sealing member 116 may provide a fluid seal over the dressing bolster 114 and a portion of the epidermis 106 of the patient. As such, the sealing member 116 may be formed from any material that allows for a fluid seal. Herein, the terms "fluid seal," or "seal," may be a seal adequate to maintain reduced pressure at a desired site given the particular reduced-pressure source or subsystem involved. The sealing member 116 may be sealed against the epidermis 106, or against a gasket or drape, by a sealing apparatus, such as, for example, a pressure-sensitive adhesive.

The sealing apparatus may take numerous forms, such as an adhesive sealing tape, drape tape, or strip; double-sided drape tape; pressure-sensitive adhesive; paste; hydrocolloid; hydrogel; or other suitable sealing device. If a tape is used, the tape may be formed of the same material as the sealing member 116 with a pre-applied, pressure-sensitive adhesive. The pressure-sensitive adhesive may be applied on a side of the sealing member 116 adapted to face the epidermis 106, such as an inward-facing side of the sealing member 116. The pressure-sensitive adhesive may provide a fluid seal between the sealing member 116 and the epidermis, and may be utilized in combination with a gasket or drape against the epidermis 106. Before the sealing member 116 is secured to the epidermis 106, removable strips or release liners that cover the pressure-sensitive adhesive may be removed.

In some embodiments, the sealing member 116 may be an elastomeric material configured to provide a fluid seal. "Elastomeric" may refer to a material having the properties of an elastomer, such as a polymeric material that has rubber-like properties. Some elastomers may have ultimate elongations greater than 100% and a significant amount of resilience. The resilience of a material may refer to the ability of the material to recover from an elastic deformation. Examples of elastomers may include, without limitation, natural rubbers, polyisoprene, styrene butadiene rubber, chloroprene rubber, polybutadiene, nitrile rubber, butyl rubber, ethylene propylene rubber, ethylene propylene diene monomer, chlorosulfonated polyethylene, polysulfide rubber, polyurethane, EVA film, co-polyester, and silicones. Further, sealing member materials may include a silicone drape, a TEGADERM^{™} drape available from 3M, an acrylic drape, such as one available from Avery Dennison, or an incise drape.

In some embodiments, the sealing member 116 may be comprised of a material including a high moisture vapor transmission rate (MVTR). The use of a high MVTR material for the sealing member 116 may permit moisture vapor to pass through the sealing member 116, external to the dressing assembly 112, while maintaining the fluid seal described herein.

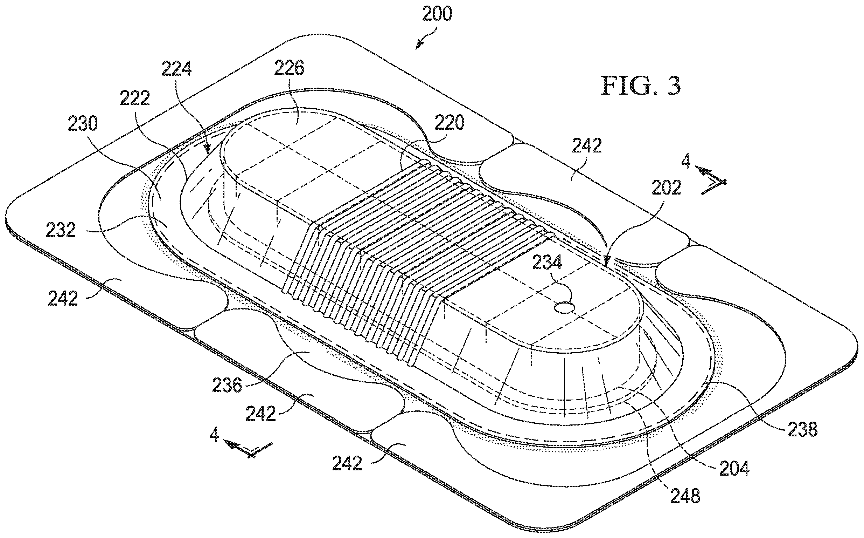

In some embodiments, the sealing member 116 may include a first sealing member portion 132 and a second sealing member portion 134. The first sealing member portion 132 may extend over or cover the first side 120 of the dressing bolster 114. The sealing member 116 may extend further to form a sealing member flange, or sealing member extension 136, which has a first side (not shown) and a second, inward-facing side (not shown). The second, inward-facing side of the sealing member extension 136 may be adapted to face the epidermis 106. An aperture (not shown) may be formed on a portion of the sealing member 116 to allow fluid communication with a conduit interface 138, which may be part of a reduced-pressure assembly 140. The aperture on the sealing member 116 may be analogous to aperture 234 depicted in FIGURE 3.

The second, inward-facing side of the sealing member extension 136 may be placed on a first side (not shown) of the second sealing member portion 134, and coupled, such as by an adhesive, a bond 135, a weld (e.g., ultrasonic or RF welding), or by cements. The first side of the second sealing member portion 134 may face away or outward from the epidermis 106. In some example embodiments, the second sealing member 134 may be positioned on and extend outward from the second side 122 of the dressing bolster 114 and be coupled to a portion of the first sealing member 132 as described. Further, in some examples, the first sealing member portion 132 and the second sealing member portion 134 may be integrally formed with one another. Further, the first sealing member portion 132 may include a plurality of bellows 142, folds, or stretch zones. The bellows 142 may provide additional drape material when needed to respond to stretching or other movement. For example, if the dressing assembly 112 is used on a joint, when the joint is flexed, the bellows 142 may provide additional drape material to facilitate such movement.

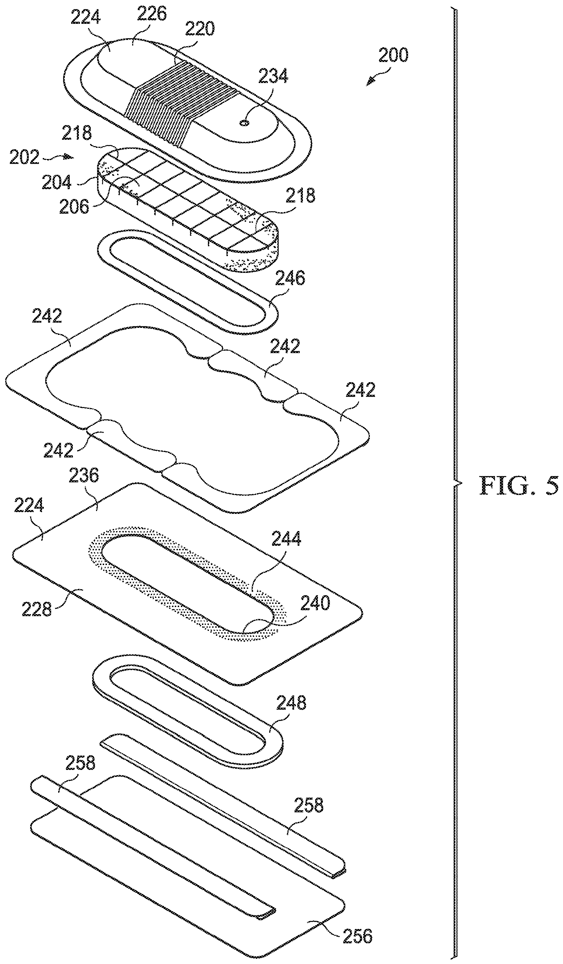

Prior to application, one or more release members (not shown) may be releasably coupled to the first side of the second sealing member portion 134. The release members may be analogous to release members 242 depicted in FIGURE 5, and may provide stiffness to assist with, for example, deployment of the dressing assembly 112. The release members may be, for example, casting paper or a film held on the first side of the second sealing member portion 134. Each release member may have a release agent disposed on a side of the release member configured to contact a component of the dressing assembly 112, such as the second sealing member portion 134, or other components described herein. In some embodiments, the release agent may be a silicone coating and may have a release factor between about 5 grams per centimeter to about 15 grams per centimeter. In some embodiments, the release factor may be between about 2 grams per centimeter to about 6 grams per centimeter. The release agent may facilitate removal of the release member by hand and without damaging or deforming the dressing assembly 112.

Release members suitable for use with the embodiments described herein may be, for example and without limitation, polyester release members specified as FRA 301(T-36) and FRA 396-T13, available from Fox River Associates, LLC of Geneva, Illinois. The polyester release members may be a polyethylene terephthalate (PET) release member as described herein. In some embodiments, the release members may have a film thickness between about 30 microns to about 70 microns. In some embodiments, the film thickness may be between about 47 microns to about 53 microns. Further, the release members may have a tensile break strength in a machine direction between about 9 kilograms per square millimeter to about 15 kilograms per square millimeter. In a transverse direction, or direction transverse to the machine direction, the release members may have a tensile break strength between about 15 kilograms per square millimeter to about 23 kilograms per square millimeter. The elongation at break of the release members in both the machine direction and the transverse direction may be between about 40 percent to about 140 percent. The release members may have a shrinkage in the machine direction between about 0.0 percent to about 2.5 percent, and a shrinkage in the transverse direction between about 0.0 percent to about 1.2 percent.

The reduced-pressure subsystem 118 may include a reduced-pressure source 144. In some example embodiments, the reduced-pressure source 144 may provide reduced pressure as a part of the treatment system 100. Further, in some examples, the reduced-pressure source 144 may be configured to be coupled in fluid communication with the dressing assembly 112. For example, the reduced-pressure source 144 may be fluidly coupled to the conduit interface 138 by a delivery conduit 148.

As used herein, the term "reduced pressure" may refer to a pressure less than the ambient pressure at a tissue site being subjected to treatment, such as the tissue site 102. The reduced pressure may be less than the atmospheric pressure. The reduced pressure may also be less than a hydrostatic pressure at a tissue site. Unless otherwise indicated, quantitative values of pressure stated herein are gauge pressures.

The reduced pressure delivered to the dressing bolster 114 may be constant or varied, patterned or random, and may be delivered continuously or intermittently. Although the terms "vacuum" and "negative pressure" may be used to describe the pressure applied to a tissue site, the actual pressure applied to the tissue site may be more than the pressure normally associated with a complete vacuum. Consistent with the use herein, unless otherwise indicated, an increase in reduced pressure or vacuum pressure may refer to a relative reduction in absolute pressure.

Continuing with FIGURE 1, the reduced-pressure source 144 is shown as having a reservoir region 146, or canister region. An interposed membrane filter (not shown), such as hydrophobic or oleophobic filter, may be interspersed between the reduced-pressure delivery conduit 148 and the reduced-pressure source 144. One or more devices, such as a representative device 150, may be fluidly coupled to the reduced-pressure delivery conduit 148. The representative device 150 may be, for example, another fluid reservoir, a collection member to hold exudates and other fluids removed, a pressure-feedback device, a volume detection system, a blood detection system, an infection detection system, a flow monitoring system, or a temperature monitoring system. Multiple representative devices 150 may be included. One or more of the representative devices 150 may be formed integrally with the reduced-pressure source 144.

The reduced-pressure source 144 may be any device for supplying a reduced pressure, such as a vacuum pump, wall suction, or other source. While the amount and nature of reduced pressure applied to a tissue site may vary according to the application, the reduced pressure may be, for example, between about -5 mm Hg (-667 Pa) to about -500 mm Hg (-66.7 kPa). In some embodiments, the reduced pressure may be between about -75 mm Hg (-9.9 kPa) to about -300 mm Hg (-39.9 kPa).

The reduced pressure developed by the reduced-pressure source 144 may be delivered through the delivery conduit 148 to the conduit interface 138. The conduit interface 138 may allow the reduced pressure to be delivered through the sealing member 116 to the dressing bolster 114. In some embodiments, the conduit interface 138 may provide fluid communication external to the sealing member 116 without the application of reduced pressure.

The reduced-pressure indicator 101 may be configured to indicate that a reduced pressure of at least of certain threshold level is being delivered to the tissue site 102. The reduced-pressure indicator 101 may be a separate unit fluidly coupled to the sealing member 116 such that reduced pressure from within the sealed space of the sealing member 116 reaches the reduced-pressure indicator 101. In some embodiments, as shown in FIGURE 1, the reduced-pressure indicator 101 may be associated with the conduit interface 138 as a part of the reduced-pressure assembly 140. When adequate reduced pressure is present, the reduced-pressure indicator 101 may be configured to assume a collapsed position. When inadequate reduced pressure is present, the reduced-pressure indicator 101 may be configured to assume a non-collapsed position.

Referring primarily to FIGURE 2, the dressing assembly 112 may include a gasket member 117. The gasket member 117 may also be referred to interchangeably as an interface seal or sealing ring 117. For example, features or characteristics of the gasket member 117 may apply to the sealing ring 117, and features or characteristics of the sealing ring 117 may apply to the gasket member 117. The gasket member 117 and the sealing ring 117 may be configured in any suitable shape to enhance or otherwise provide a fluid seal around the tissue site 102, such as the incision 104. For example, the epidermis 106 may have recesses, cracks, wrinkles, or other discontinuities on a surface of the epidermis 106 that may cause leaks. Moreover, folds, buckles, wrinkles, or other discontinuities may form in the sealing member 116 and cause leaks. The gasket member 117 and the sealing ring 117 may help seal any such skin or sealing member discontinuities around the tissue site 102.

The gasket member 117 or the sealing ring 117 may be adapted to be positioned between the dressing assembly 112 and the epidermis 106 and/or the tissue site 102. In some examples, the gasket member 117 may be positioned on, coupled to, or directly coupled to the second side 122 of the dressing bolster 114 or the second side 128 of the comfort layer 124. The gasket member 117 or the sealing ring 117 may be formed, as an illustrative example, by applying or bonding a sealing material around a perimeter or circumference of a portion of the dressing assembly 112. Although the sealing material of the gasket member 117 or the sealing ring 117 may be configured in the shape of a ring in some embodiments, other shapes are suitable, and may include, without limitation, circles, squares, rectangles, discontinuous shapes, continuous shapes, irregular shapes, linear shapes, other shapes or portions that overlap one another, or combinations thereof. The sealing material may include hydrocolloids, hydrogels, silicone polymers (both crosslinked and uncrosslinked gels), and natural gums (xanthan, guar, cellulose). The sealing material may include other soft polymer gels, such as, for example, those based on polyurethanes, polyolefin gels, and acrylics.

In some embodiments, the gasket member 117 or the sealing ring 117 may be deployed by hand or extruded from an applicator, such as a syringe, to form a ring or other shape prior to application of the dressing assembly 112 to the tissue site 102. Sealing materials suitable for application by extrusion may include, without limitation, water soluble gums such as xanthan, guar, or cellulose, and thick greases, such as silicones. In another embodiment, the sealing ring 117 may be bonded in any suitable manner, such as, for example, by a heat bond, to the second, inward facing side 128 of the comfort layer 124 during manufacture of the dressing assembly 112. In at least this manner, the sealing ring 117 may be adapted to be positioned between the comfort layer 124 and the epidermis 106 and/or the tissue site 102.

In some embodiments, the gasket member 117 or the sealing ring 117 may include an absorbent. For example, the sealing ring 117 may be a hydrocolloid comprising an absorbent, such as carboxy methyl cellulose (CMC). The absorbent may permit the sealing ring 117 to absorb fluid from the tissue site 102 in addition to enhancing the fluid seal around the tissue site 102. The sealing ring 117 including the absorbent may enhance the ability of the dressing assembly 112 to manage and direct fluid away from the tissue site 102 for keeping the tissue site 102 dry. For example, the dressing bolster 114 may have a thickness between the first side 120 and the second, inward-facing side 122 of the dressing bolster 114. The thickness of the dressing bolster 114 may define at least a portion of a thickness of the dressing assembly 112. The sealing ring 117 may be adapted to be positioned between the dressing assembly 112 and the tissue site 102, as described above, and around or surrounding a circumference of the tissue site 102. Relative to the dressing assembly 112, the sealing ring 117 or the gasket member 117 may be positioned, for example, around, on, or at the lateral edges of the dressing bolster 114 and/or the comfort layer 124. Further, the sealing ring 117 or the gasket member 117 may extend beyond a lateral edge of the dressing bolster 114 and the comfort layer 124. Further, the sealing ring 117 may be positioned around or surrounding a circumference of the dressing bolster 114 and/or the comfort layer 124. Further, the sealing ring 117 may be positioned around at least a portion of the dressing bolster 114 or the comfort layer 124 that is configured to be positioned directly against or in direct contact with the tissue site 102. At least a portion of the dressing bolster 114 and/or the comfort layer 124 may be exposed and configured to be positioned directly against the tissue site 102 when the sealing ring 117 is positioned on the dressing assembly 112. Further, in such embodiments, the sealing ring 117 may surround the exposed portion of the dressing bolster 114 and/or the comfort layer 124.

The absorbent in the sealing ring 117 may wick or draw fluid in a lateral direction within the dressing assembly 112, normal to the thickness of the dressing bolster 114, and toward the lateral edges of the dressing bolster 114 for absorption in the sealing ring 117. Thus, fluid from the tissue site 102 may be wicked or otherwise drawn in a lateral direction along the surface of the tissue site 102 toward the lateral edges of the dressing bolster 114 and into the sealing ring 117. Further, fluid from the tissue site 102 may also flow through the thickness of the dressing assembly 112 and the dressing bolster 114 at least by operation of the manifold material comprising the dressing bolster 114, described above.

Referring now primarily to FIGURES 3-5, depicted is a portion of an illustrative embodiment of a treatment system 200 suitable for treating, for example, a linear wound, area wound, a graft, or other wound. FIGURES 3-5 depict the treatment system 200 in a pre-deployment state. The treatment system 200 may include a dressing assembly 202, and the dressing assembly 202 may include a dressing bolster 204. The dressing bolster 204 has a first side 206 and a second, inward-facing side 208. The dressing bolster 204 may be formed from any suitable bolster material, or manifold material, as previously referenced in connection with the dressing bolster 114. A comfort layer 210, which has a first side 212 and a second, inward-facing side 214, may be coupled, such as, for example, by a heat bond 216 or other suitable technique to the second, inward-facing side 208 of the dressing bolster 204.

The comfort layer 210 may be any material that helps prevent skin irritation and discomfort while allowing fluid transmission through the comfort layer 210. Suitable materials for the comfort layer 210 have been mentioned in connection with the comfort layer 124 of FIGURES 1-2. In some embodiments, the comfort layer 210 may include antimicrobial substances, such as silver. Further, in some embodiments, the comfort layer 210 may be made as a breathable, dry layer.

In some illustrative embodiments, the dressing bolster 204 may include a plurality of flexibility notches 218. The flexibility notches 218 may extend partially through or completely through the dressing bolster 204. The flexibility notches 218 may be lateral notches, or lateral cuts, in the dressing bolster 204. The flexibility notches 218 may also be one or more longitudinal notches, longitudinal cuts, or other cuts. The cuts may be made using a saw, a notched blade, a hot knife, or other device. The flexibility notches 218 may enhance the flexibility of the dressing bolster 204. The enhanced flexibility may be particularly useful when the dressing assembly 202 is applied over a joint or other area of movement on a patient. For example, if the dressing bolster 204 is used on a knee, the dressing bolster 204 may need to flex or extend as much as 100 % or more. The flexibility notches 218 may provide such flexibility.

The dressing bolster 204 may have lateral edges 205 that are orthogonal with respect to the second, inward-facing side 208 of the dressing bolster 204. The lateral edges 205 may also have a shape, such as, for example, a beveled, angled, or rounded shape. The lateral edges 205, when angled, may be between about 10 degrees to about 90 degrees with respect to the second, inward-facing side 208 of the dressing bolster 204. The shaped lateral edges 205 may reduce shear stress between an epidermis of a patient and the dressing bolster 204. Other dimensions, steps, and processes may be used.

In some illustrative embodiments, the dressing bolster 204 may be manufactured from a foam block of GRANUFOAM^{™} material. The GRANUFOAM^{™} material may be, for example, a foam block having the dimensions of 1.21 meters x 1.8 meters x 0.5 meters. The foam block may be cut to have a 19 millimeter height, and a saw may be used to form lateral grooves, such as the flexibility notches 218, in the foam block. A dry layer, such as the comfort layer 210, may be laminated or otherwise attached to the second, inward facing side 208 of the dressing bolster 204. The foam block may be cut, for example, utilizing a die cutter to form a plurality of individual dressing bolsters 204.

A sealing subsystem 222 may provide a fluid seal over the dressing assembly 202 and at least a portion of an epidermis of a patient. The sealing subsystem 222 may include a sealing member 224. The sealing member 224 may be formed with an upper drape portion or first sealing member portion 226 and a lower drape portion or second sealing member portion 228. The first sealing member portion 226 may extend over or cover the first side 206 of the dressing bolster 204 to form a drape flange, or drape extension 230. The drape extension 230 has a first side 232 and a second, inward-facing side 233. The second, inward-facing side 233 of the drape extension 230 may be adapted to face a tissue site of a patient as described above. An aperture 234 may be formed on the first sealing member portion 226. The aperture 234 may provide fluid communication with a conduit interface (not shown). The conduit interface may be analogous to the conduit interface 138 in FIGURE 1.

The second sealing member portion 228 may have a first side 236 and a second, inward-facing side 237 adapted to face a tissue site as described above. The second, inward-facing side 233 of the drape extension 230 may be placed on the first side 236 of the second sealing member portion 228, and may be coupled to the first side 236 by an attachment device 238. The attachment device 238 may be, for example, an adhesive, a bond, a weld (e.g., ultrasonic or RF weld), cements, stitching, staples, or other coupling device. The second sealing member portion 228 may include an attachment apparatus on the second, inward-facing side 237 as described below. The second sealing member portion 228 may also include a treatment area aperture 240, depicted in FIGURE 5, that may be adapted to permit fluid communication through the second sealing member portion 228 and, for example, between a tissue site and the dressing bolster 204. The treatment area aperture 240 may also provide an opening for at least a portion of the dressing bolster 204, or the comfort layer 210, to be positioned directly against an epidermis and/or a tissue site of a patient.

The first sealing member portion 226 may include a plurality of folds 220 or bellows to facilitate movement as described above. The folds 220 may allow the first sealing member portion 226 to expand. For example, if the dressing assembly 202 is used on a joint, when the joint is flexed, additional drape material from the folds 220 may be released to facilitate movement of the first sealing member portion 226. The folds 220 may also be formed as ridges having the cross-sectional shape of an accordion that provides additional drape material when flattened or stretched, for example.

One or more release members 242 may be releasably coupled to the first side 236 of the second sealing member portion 228, such as, for example, with an adhesive (not shown) applied on at least a portion of the first side 236. Four of the release members 242 are shown in the illustrative embodiment of FIGURE 3. The release members 242 may provide stiffness to the second sealing member portion 228, and may cover the adhesive or other attachment apparatus to provide a grasping surface during deployment of the dressing assembly 202. The release members 242 may be casting paper or a film held on the first side 236 of the second sealing member portion 228.

The first side 236 of the second sealing member portion 228 may include an adhesive 244 adapted to retain the second side 208 of the dressing bolster 204 against the second sealing member portion 228 during assembly and usage. A center release member 246 may cover and protect the adhesive 244 prior to assembly. The release members 242 that may provide stiffness to the sealing member 224 during deployment may be positioned outboard of the adhesive 244 on the first side 236 of the second sealing member portion 228.

The dressing assembly 202 may include a sealing ring 248. Analogous to the sealing ring 117, the sealing ring 248 may help seal any wrinkles or discontinuities in the epidermis or drape that might otherwise cause leaks. Also analogous to the sealing ring 117, the sealing ring 248 may also be referred to interchangeably as an interface seal or a gasket member 248. For example, features or characteristics of the gasket member 248 may apply to the sealing ring 248, and features or characteristics of the sealing ring 248 may apply to the gasket member 248. Further, the previously described features of the sealing ring 117 or the gasket member 117 associated with the dressing assembly 112 may apply by analogy to the sealing ring 248 or the gasket member 248 associated with the dressing assembly 202.

The sealing ring 248 or the gasket member 248 may be, for example, positioned to cover a portion of the second, inward-facing side 237 of the second sealing member portion 228. The sealing ring 248 or the gasket member 248 may be coupled directly to the dressing assembly 202, or coupled with an optional sealing-ring attachment device 249, such as an acrylic adhesive, cement, or other coupling device. In other embodiments, the sealing ring 248 or the gasket member 248 may be coupled to the second inward-facing side 208 of the dressing bolster 204, and/or to an adjacent layer, such as the comfort layer 210.

The sealing ring 248 or the gasket member 248 may straddle an edge of the dressing bolster 204, or otherwise extend beyond an edge of the dressing bolster 204, as depicted in FIGURE 4. In some embodiments, the sealing ring 248 or the gasket member 248 may be coupled to a portion of the sealing member 224, such as the first sealing member 226 and/or the second sealing member 228. In some embodiments, the dressing bolster 204 may entirely overlap the sealing ring 248 or the gasket member 248 as suggested in FIGURE 11. While reference is made to a "ring," discrete members, including linear members, may make up the sealing ring 248 or the gasket member 248.

The sealing ring 248 may comprise a sealing material, such as, for example, any of the sealing materials previously described in connection with the sealing ring 117, or other material that provides initial tack between the dressing assembly 202 and an epidermis of a patient. Further, the sealing ring 248 may have a durometer, such as a material softness or hardness, between about 20 Shore 00 to about 90 Shore OO. In some embodiments, the durometer of the sealing ring 248 may be between about 70 Shore 00 to about 80 Shore OO. The sealing ring 248 may have a modulus of elasticity that falls between the modulus of elasticity of the second sealing member portion 228 and the modulus of elasticity of a tissue site and/or epidermis of a patient. As shown in FIGURE 4, the sealing ring 248 may have a thickness 250 and a width 252. The thickness 250 of the sealing ring 248 may be between about 0.3 millimeters to about 2.5 millimeters. In some embodiments, the thickness 250 may be between about 0.7 millimeters to about 1.25 millimeters. The width 252 of the sealing ring 248 may be between about 10 millimeters to about 30 millimeters. Other dimensions are possible. In some illustrative embodiments, the thickness 250 may be about 0.7 millimeters and the width 252 may be about 20 millimeters. Further, in some embodiments, the width 252 of the sealing ring 248 may extend beyond an edge of the dressing bolster 204 by about 10 millimeters and overlap the dressing bolster 204 by about 10 millimeters.

In some embodiments, the second sealing member portion 228 may have a thickness 229 between about 0.178 millimeters to about 0.254 millimeters, or about 7 mils to about 10 mils. The ratio of the sealing ring thickness 250 to the sealing member thickness 229 may be between about 2.75 to about 7.03.

The sealing ring 248 may include fenestrations or apertures. In some embodiments, the sealing ring 248 may comprise a patterned sealing material on the second, inward-facing side 214 of the comfort layer 210, or on the second, inward-facing side 208 of the dressing bolster 204. The pattern may be, for example, spaced islands, crossing lines of sealing material, or any other suitable pattern.

The sealing ring 248 may function as a two-sided gasket that may provide a seal between the dressing assembly 202 and a tissue site and/or epidermis of a patient. For example, the sealing ring 248 may provide a seal between the dressing bolster 204, the comfort layer 210, or the second sealing member portion 228 and a tissue site and/or epidermis of a patient. The sealing ring 248 may absorb perspiration or other fluids from a tissue site. Further, the sealing ring 248 may help distribute shear forces created, for example, by the application of reduced pressure at the interface of the dressing bolster 204 and a tissue site and/or epidermis of a patient.

As shown in FIGURE 4, a portion of the second, inward-facing side 237 of the second sealing member portion 228 may be covered with a sealing apparatus or device 254, such as an adhesive. With reference to FIGURES 4 and 5, when in the pre-deployment state, the sealing device 254 may be covered by a bottom release member 256 and side release members 258.

The bottom release member 256 may cover and protect, for example, the sealing device 254 and the sealing ring 248. The side release members 258 may also cover and protect the sealing device 254. Similar to the release members 242, the side release members 258 may provide a grasping surface for a user to facilitate deployment of the dressing assembly 202. The release members 242, the bottom release member 256, and /or the side release members 258 may be comprised of a polar semi-crystalline polymer, such as, for example, polyethylene terephthalate (PET). Use of a polar semi-crystalline polymer for the release members 242, the bottom release member 256, and /or the side release members 258 may substantially preclude wrinkling or other deformation of the dressing assembly 202. Any deformation of the release members 242, the bottom release member 256, and/or the side release members 258 may cause wrinkling or deformation of a component of the dressing assembly 202. The polar semi-crystalline polymer is highly orientated and resistant to softening, swelling, or other deformation that may occur when brought into contact with components of the dressing assembly 202, or when subjected to temperature or environmental variations, or sterilization. Thus, for example, when the polar semi-crystalline polymer is used in combination with the hydrocolloid described above for the sealing ring 248, the polar semi-crystalline polymer may not deform when in contact with the compounding ingredients of the hydrocolloid. In some embodiments, the release members 242, the bottom release member 256, and/or the side release members 258 may be configured to resist deformation when exposed to temperature variations between about 40 degrees Celsius to about 60 degrees Celsius, and gamma sterilization doses between about 25kGy to about 45 kGy.

Continuing with FIGURES 3-5, according to an illustrative embodiment of operation, the bottom release member 256 may be removed to expose the sealing device 254 on the second, inward-facing side 237 of the second sealing member portion 228. Removal of the bottom release member 256 may also expose a second, inward-facing surface 247 of the sealing ring 248. The sealing device 254 and/or the second, inward-facing surface 247 of the sealing ring 248 may be placed against a portion of an epidermis of a patient and around a tissue site that may include a linear wound as described above. The side release members 258 may be removed after applying the second sealing member portion 228. Similarly, the release members 242 on the first side 236 of the second sealing member portion 228 may be removed after applying the second sealing member portion 228. A conduit interface may be coupled to the aperture 234 in the first sealing member portion 226, and reduced pressure may be delivered to the dressing assembly 202.

Regarding the manufacture of the systems and components described above, in applying and coupling a sealing member to a dressing bolster, a press may be utilized to remove any wrinkles in the sealing member. Further, the medical bolster material of the shaped dressing assembly may be cut using a die cutter, or by hand with a router.

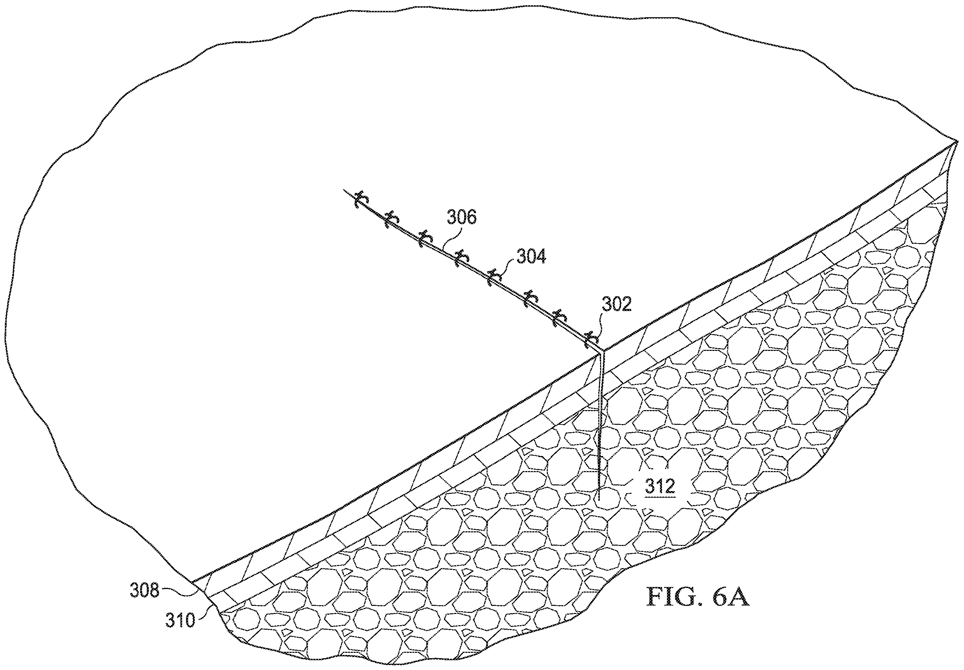

Referring now primarily to FIGURES 6A-6C, presented is another illustrative embodiment of a portion of a treatment system 300. FIGURES 6A-6C depict the treatment system 300 assembled in stages at a tissue site, such as a linear wound 306. In FIGURE 6A, a closure device 302, such as, for example, stitches 304, close the linear wound 306. Other closure devices 302, such as epoxy or staples may be utilized to close the linear wound 306. The linear wound 306 may include a portion through an epidermis 308, dermis 310, and subcutaneous tissue 312 of a patient.

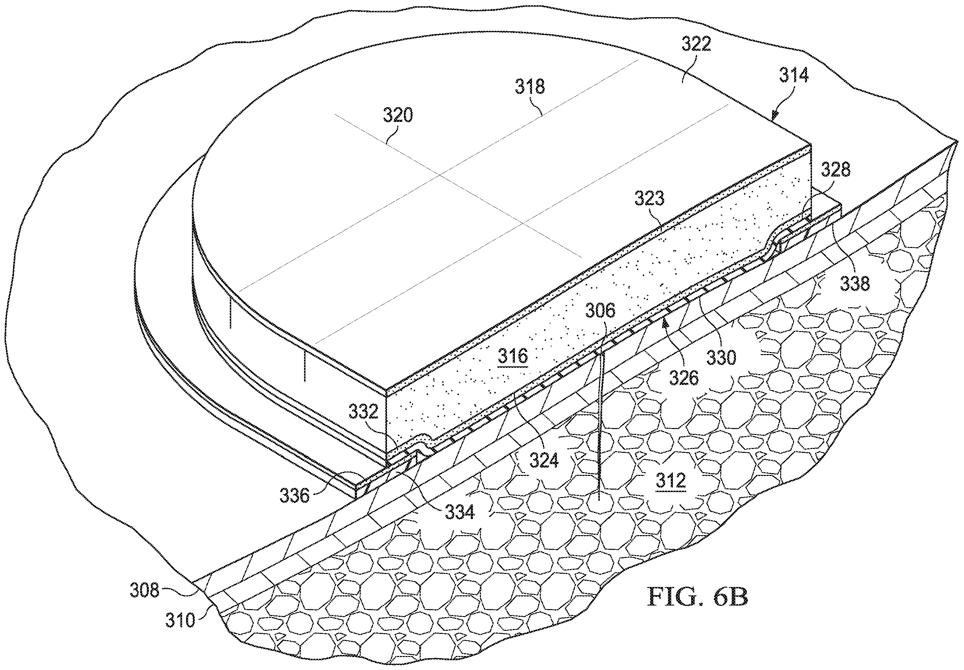

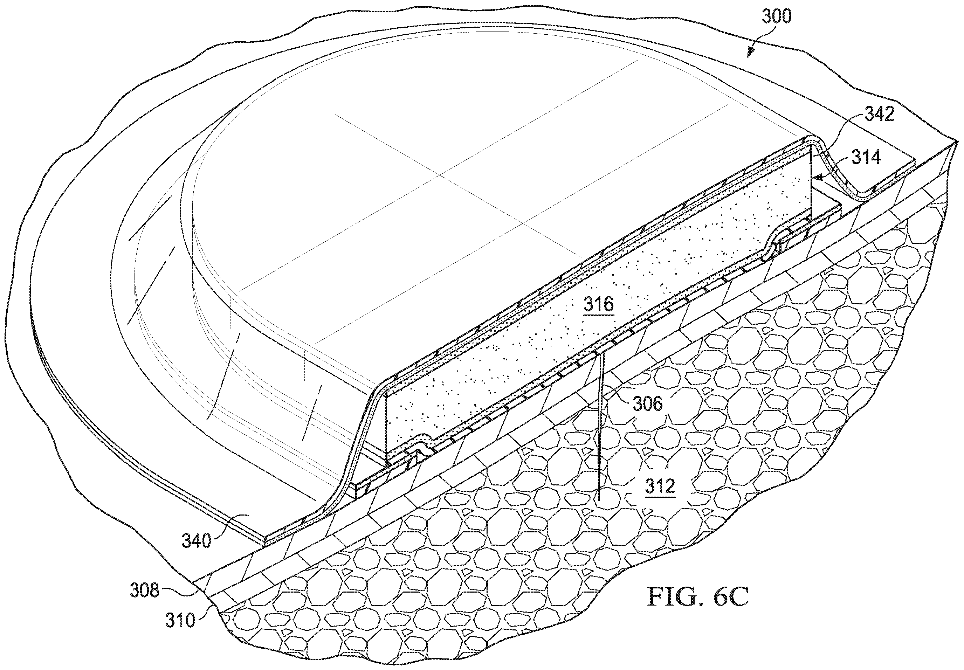

Referring now to FIGURE 6B, after the linear wound 306 is closed or prepared as described above, a dressing assembly 314 may be disposed proximate to the linear wound 306. The dressing assembly 314 may include a dressing bolster 316. The dressing bolster 316 may be formed from the bolster or manifold materials previously mentioned. The dressing bolster 316 may include a plurality of lateral notches 318 and one or more longitudinal notches 320. The dressing bolster 316 has a first side 322 and a second, inward-facing side 324. The first side 322 may include an adhesive layer 323. The adhesive layer 323 may help secure a sealing member 340 thereto, as shown in FIGURE 6C.

The dressing assembly 314 may include a comfort layer 326. The second, inward-facing side 324 of the dressing bolster 316 may be covered with the comfort layer 326. The comfort layer 326 has first side 328 and a second, inward-facing side 330. The first side 328 of the comfort layer 326 may be coupled by an attachment device 332, such as, for example, a heat bond, adhesive, weld, or other attachment device, to the second, inward-facing side 324 of the dressing bolster 316.

The dressing assembly 314 may include a sealing ring 334. The sealing ring 334 may be coupled, at least in part, to the second, inward-facing side 330 of the comfort layer 326. The sealing ring 334 may be analogous to the sealing ring 117 of FIGURE 2 and the sealing ring 248 of FIGURES 3-5. Further, the sealing ring 334 may be referred to interchangeably as a gasket member 334 analogous to the gasket member 117 and the gasket member 248.

The sealing ring 334 may comprise any of the sealing materials previously described in connection with the sealing ring 117 and the sealing ring 248. The sealing ring 334 may adhere directly to the comfort layer 326, or may be coupled with a sealing-ring attachment device 336 to the comfort layer 326. The sealing-ring attachment device 336 may be, for example, acrylic adhesive, cement, or other suitable attachment device. The sealing ring 334 and/or the sealing ring attachment device 336 may be co-extensive with the comfort layer 326, or may extend beyond a lateral edge of the comfort layer 326 and the dressing bolster 316.

Prior to application, a second, inward-facing surface 338 of the sealing ring 334 may be covered by a release member or release liner (not shown). When the release liner is removed, the sealing ring 334 may be centered about the linear wound 306 for deployment. A release member or release liner (not shown) may also temporarily cover a portion of the sealing ring 334 and/or sealing ring attachment device 336 to provide a grasping surface during deployment of the dressing assembly 314. The release liner or release member covering, for example, the sealing ring 334, the sealing ring attachment device 336, and/or other components of the dressing assembly 314 may be analogous to the release members 242, 256, and 258 of FIGURES 3-5. For example, the release members may be positioned on the dressing assembly 314 analogous to the release members 242, 256, and 258. Further, the release members on the dressing assembly 314 may be comprised of any of the materials previously described for the release members 242, 256, and 258, such as, for example, a polar semi-crystalline polymer or polyethylene terephthalate (PET). As described above, use of a polar semi-crystalline polymer such as PET as a release member on the dressing assembly 314 may substantially preclude deformation of the dressing assembly 314. In another embodiment, the sealing ring 334 may be separately applied around the linear wound 306 before the dressing bolster 316 is applied thereto.

Referring now to FIGURE 6C, a sealing member 340 may be disposed over the dressing assembly 314 and a portion of the epidermis 308 to form a sealed space 342 between the dressing assembly 314 and the linear wound 306. Analogous to the aperture 234 in FIGURE 3, an aperture (not shown) may be formed or preformed in the sealing member 340. A conduit interface (not shown), analogous to conduit interface 138 described above, may be coupled to the sealing member 340 to provide fluid communication with the sealed space 342 through the aperture. Further, a reduced-pressure source (not shown), analogous to the reduced-pressure source 144 in FIGURE 1, may be coupled to the conduit interface to provide reduced pressure to the sealed space 342 to treat the linear wound 306. A delivery conduit (not shown), analogous to the delivery conduit 148 in FIGURE 1, may be utilized for coupling the reduced pressure source to the conduit interface. Reduced pressure may be applied to the tissue site, such as the linear wound 306, and fluid may be extracted from the tissue site and into the dressing assembly 314. The fluid from the tissue site may be absorbed into the sealing ring 334. The fluid from the tissue site may be wicked or otherwise communicated in a lateral direction within the dressing assembly 314 toward the sealing ring 334.

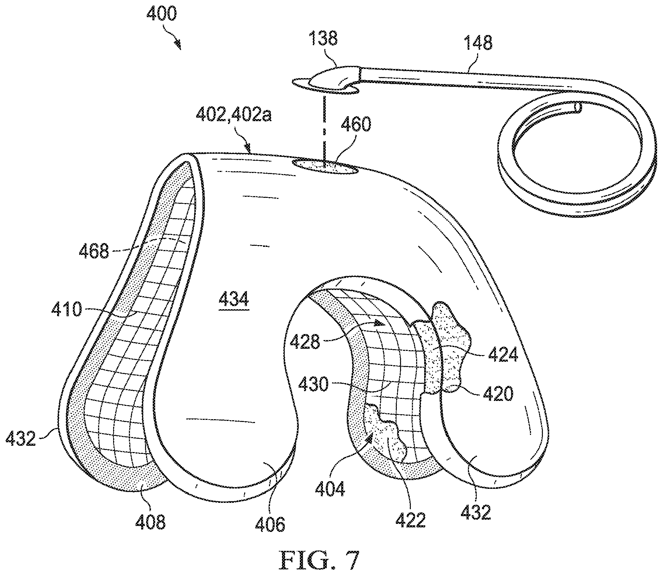

Referring now to FIGURE 7, illustrated is another example embodiment of a treatment system 400 and a dressing assembly 402 according to this disclosure. The system 400 may include similar or analogous features as the treatment system 100, 200, 300, and the dressing assembly 402 may include similar or analogous features as the dressing assembly 112, 202, 314. For brevity, features of the system 100, 200, 300 and the dressing assembly 112, 202, 314 not specifically described in connection with the system 400 and the dressing assembly 402 may be applied analogously or included in the system 400 and the dressing assembly 402, respectively. Further, features of the treatment system 400 and the dressing assembly 402 may be added or removed by persons of skill to suit various applications.

In some examples, the dressing assembly 402 may include a dressing bolster 404 and a sealing skin 406. Further, the dressing assembly 402 may optionally, additionally or alternatively include a gasket member 408 similar or analogous to the gasket member 117, 248, 334. Further, the dressing assembly 402 may optionally, additionally or alternatively include a tissue interface 410 similar or analogous to the tissue interface layer 124, 210, 326.

The dressing bolster 404 may include similar or analogous features as the previously described dressing bolster 114, 204, and 316. For example, the dressing bolster 404 may include a first side 420, an opposing second side 422, and an edge 424 between the first side 420 and the second side 422 of the dressing bolster 404. Further, the dressing bolster 404 may include or be formed of, without limitation, a porous foam, an open-cell foam, or any of the materials previously described for the dressing bolster 114, 204, and 316.

The second side 422 of the dressing bolster 404 may be configured to be positioned in fluid communication with the tissue site 102. Further, the second side 422 of the dressing bolster 404 may be configured to face the tissue site 102 or to be positioned in contact with the tissue site 102. In some examples, the tissue interface 410, which may be similar or analogous to the tissue interface 124, 210, 326, may be coupled to or at the second side 422 of the dressing bolster 404 and configured to be positioned in contact with the tissue site 102. The tissue interface 410 may include or be formed of similar or analogous materials as described for the tissue interface 124, 210, 326, such as, without limitation, a woven material, a non-woven material, a polyester knit material, a fenestrated film, a felted foam, and a foam skin.

The dressing bolster 404 of the invention has a contoured shape and may be referred to as a contoured bolster 404 with the same reference numeral. Further, features associated with the dressing bolster 404 may apply to the contoured bolster 404, and features associated with the contoured bolster 404 may apply to the dressing bolster 404. The contoured bolster or dressing bolster 404 includes a tissue housing 428 on the second side 422 of the dressing bolster 404. The tissue housing 428 includes a three-dimensional contact surface 430 configured to receive at least a portion of the tissue site 102 and to contact the tissue site 102 in three-dimensions. In some examples, the three-dimensional contact surface 430 may include a concave shape. Further, in some examples, the dressing bolster 404 may include one or more protrusions or wings 432 that extend outward from a periphery the dressing assembly 402 or form part of the periphery of the dressing assembly 402 to increase a treatment area, sealing area, or the conformability of the dressing assembly 402 at the tissue site 102. In some examples, one or more of the wings 432 may form part of the edge 424 of the dressing bolster 404. Further, in some examples, the size of the dressing assembly 402 may range from about 75 millimeters to about 205 millimeters in diameter at an opening of the tissue housing 428. Further, in some examples, a thickness of the dressing bolster 404 at the edge 424 may be between about 14 millimeters to about 20 millimeters.

The sealing skin 406 may cover at least a portion of the first side 420 of the dressing bolster 404. Further, the sealing skin 406 may be liquid impermeable and may be integrally formed with the dressing bolster 404. In some examples, the sealing skin 406 may include or may be a liquid impermeable surface 434 formed on and from a portion of the porous foam of the dressing bolster 404. In some examples, the sealing skin 406 may cover the first side 420 of the dressing bolster 404, the edge 424 of the dressing bolster 404 between the first side 420 and the second side 422 of the dressing bolster 404, and a portion of the second side 422 of the dressing bolster 404.

In some examples, the sealing skin 406 may be formed as a film skin on a surface of the dressing bolster 404 as part of a manufacturing process associated with the dressing bolster 404. For example, a pre-polymer mixture configured to produce a foam may be placed or injected into a mold to form at least part of the dressing assembly 402 or the dressing bolster 404. In some embodiments, the pre-polymer mixture may include ingredients, such as isocyanate and polyol, for forming a polyurethane foam when mixed with water. Additional examples may include ingredients for forming a polyethylene foam when mixed with low-boiling-point liquids or pressurized gases, and accordingly the pre-polymer mixture may comprise molten polyethylene and low-boiling- point liquids such as fluorocarbons, pressurized gasses such as nitrogen, and/or chemical blowing agents such as citric acid and carbonate or bicarbonate salts. As the ingredients of the mixture react within the mold, a foam may be generated and a film may also develop on one or more surfaces of the foam. For example, as the foam contacts one or more walls of the mold, the relative temperature of the portion, or surface, of the foam contacting the wall of the mold may be reduced, thereby retarding or stopping the reaction of ingredients forming the foam. As a result, an integral film skin may be formed on the surfaces of the foam contacting the wall of the mold, with the integral film skin being a portion of foam having a very high density, for example, a much higher density than the remainder of the generated foam. In some instances, the integral film skin may have a sufficiently high density to essentially be free of pores. In some examples where the foam is a polyurethane foam, the integral film skin may be or form a polyurethane elastomer. The integral film skin may provide the sealing skin 406 as described herein.

In other examples, the sealing skin 406 may be or may include a semi-rigid polymer shell (not shown) molded into a shape configured to be contoured to or configured to conform to a multi-dimensional or multi-surface tissue site, such as, without limitation, a shoulder, knee, elbow, breast, or similar anatomical location. The semi-rigid polymer shell may include or be formed of a liquid impermeable material. Further, in such an embodiment, the semi-rigid polymer shell may be configured to support and to maintain the position of the dressing bolster 404 in a contoured shape along with other components or features of the dressing assembly 402 described herein.

Further, in other examples, the sealing skin 406 may be a surface of the dressing bolster 404 that has been treated support and to maintain the position of the dressing bolster 404 in a contoured shape and/or to have liquid impermeable properties. In such an example, the dressing bolster 404 may be treated by various methods including, without limitation, the application of a coating or a felting process.

As described herein, the terms "felting" or "felted" may refer to a porous material or a portion of a porous material, such as the dressing bolster 404, which has been treated or modified to impart a desired property to the porous material. For example, the porous material may be a foam treated or modified to have a desired porosity or density. Felting may be performed by any known methods, which may include applying heat and pressure to a porous material or foam material. Such methods may include compressing the porous material between one or more heated platens or dies (not shown) for a specified period of time and at a specified temperature. The direction of compression may be along the thickness of the porous material.

The period of time of compression may range from 10 minutes up to 24 hours, though the time period may be more or less depending on the specific type of porous material used. Further, in some examples, the temperature may range between 120°C to 260°C. Generally, the lower the temperature of the platen, the longer a porous material must be held in compression. After the specified time period has elapsed, the pressure and heat will form a felted structure or surface on or through the porous material or a portion of the porous material. The felted structure may be comparatively smoother than any unfinished or non-felted surface or portion of the porous material. Further, the pores in the felted structure may be smaller than the pores throughout any unfinished or non-felted surface or portion of the porous material. In some examples, the felted structure may be applied to all surfaces or portions of the porous material. Further, in some examples, the felted structure may extend into or through an entire thickness of the porous material such that the all of the porous material is felted.

Felting may be expressed as a ratio of the uncompressed thickness or the initial thickness of the porous material to the compressed or final thickness of the porous material after the felting process has taken place. For example, a felting ratio of 1:3 compresses the porous material to one-third of the uncompressed or the initial thickness of the porous material. A felting ratio of 1:7 compresses the porous material to one-seventh of the uncompressed or the initial thickness of the porous material. In some embodiments, the compressed thickness of the porous material may be less than one-tenth, one-ninth, one-eighth, one-seventh, one-sixth, one-fifth, one-fourth, one-third, or one-half of the uncompressed or the initial thickness of the porous material. When the felting process is complete, the porous material may retain a particular shape or contour into which it was felted, and the felted portion may restrict or prevent fluid communication. Further, in some embodiments, an optional auxiliary drape, cover, or sealing member, similar or analogous to the sealing member 116, may be applied over the dressing bolster 404 in applications where additional sealing capability is desired.

The gasket member 408 may be configured to create or to enhance formation of a sealed space between the sealing skin 406 and the tissue site 102 analogous to the previously described sealed space 342. The dressing bolster 404 may be configured to be positioned in the sealed space at the tissue site 102 when the dressing assembly 402 is deployed at the tissue site 102. In some examples, the gasket member 408 may be positioned on a portion of the second side 422 of the dressing bolster 404. Further, the gasket member 408 may be positioned around a circumference of the dressing bolster 404 and configured to surround the tissue site 102. Further, the gasket member 408 may be positioned at the edge 424 of the dressing bolster 404. A portion of the sealing skin 406 may be positioned between the second side 422 of the dressing bolster 404 and the gasket member 408. Further, in some examples, the gasket member 408 may be coupled to a portion of the tissue interface 410, and the portion of the tissue interface 410 may be positioned between the second side 422 of the dressing bolster 404 and the gasket member 408. Further, in some examples, the gasket member 408 may include or may be, without limitation, a hydrocolloid or any of the previously described materials for the gasket member 117, 248, 334.

The reduced-pressure source 144, previously described and illustrated in FIGURE 1, may be configured to be coupled in fluid communication with the dressing bolster 404 through the sealing skin 406 similar or analogous to the previously described sealing member 116, 224, 340. In some examples, the dressing assembly 402 may include the reduced pressure interface 138 coupled at the first side 420 of the dressing bolster 404 and in fluid communication with the dressing bolster 404 through the sealing skin 406. In some examples, the reduced pressure interface 138 may be coupled in fluid communication with the reduced pressure source 144 with the delivery conduit 148 as previously described.

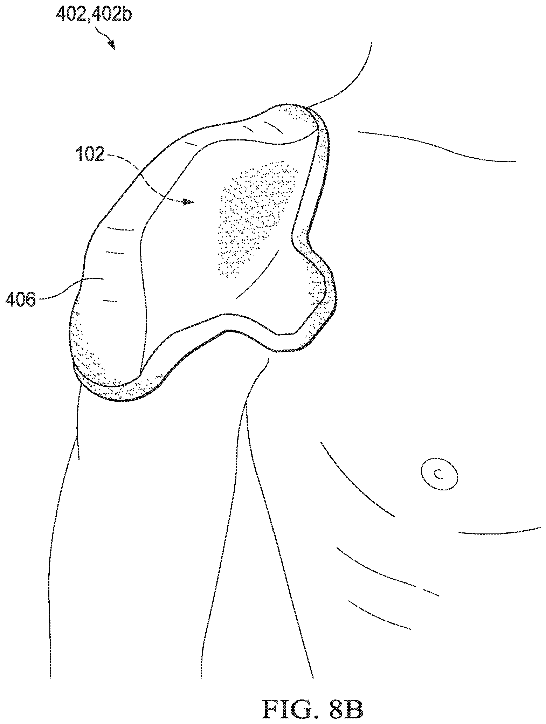

Referring to FIGURE 8A, shown is the dressing assembly 402, illustrated as a dressing assembly 402a, deployed at a tissue site, which in this example is a shoulder of a patient. In other examples, the dressing assembly 402 may have any contoured, non-planar shape suitable for treating a variety of tissue sites having different three-dimensional or complex geometries, such as, without limitation, a shoulder, ankle, breast, hand, or sacral region of a patient. For example, referring to FIGURE 8B, the dressing assembly 402 is illustrated as a dressing assembly 402b deployed over a shoulder of a patient, but the dressing assembly 402b includes a different shape than the previously described dressing assembly 402a.

In some example embodiments, a method for treating the tissue site 102 may include disposing the dressing assembly 402 according to this disclosure proximate to the tissue site 102. For example, the dressing assembly 402 may include the contoured bolster 404, the sealing skin 406, and the tissue housing 428 as described herein. The sealing skin 406 may be formed integrally with at least a portion of the first side 420 of the contoured bolster 404, and the tissue housing 428 may be formed on the second side 422 of the contoured bolster 404. The method may include inserting at least a portion of the tissue site 102 into the tissue housing 428. Further, the method may include forming a sealed space between the sealing skin 406 and the tissue site 102, and positioning the contoured bolster 404 in the sealed space. Forming the sealed space may occur without the application of a cover, drape, or other sealing device over the dressing assembly 402. Further, the method may include distributing reduced pressure to the tissue site 102 through the contoured bolster 404.

As described herein, the tissue housing 428 may include the three-dimensional contact surface 430 configured to receive at least a portion of the tissue site 102. In some examples, inserting at least a portion of the tissue site 102 into the tissue housing 428 may include contacting at least the portion of the tissue site 102 in three-dimensions. Further, in some examples, the method may include coupling the reduced pressure interface 138 at the first side 420 of the contoured bolster 404 in fluid communication with the contoured bolster 404 through the sealing skin 406. The reduced pressure interface 138 may be fluidly coupled to the reduced-pressure source 144 as described herein.

The configuration of the dressing assembly 402 including the integral sealing skin 406 does not require a cover, drape, or other sealing device to be applied over the dressing assembly 402 to form a sealed space, for example, similar or analogous to the sealed space 342. Accordingly, the construction of the dressing assembly 402 itself and the methods of treatment using the dressing assembly 402 are simplified, requiring fewer components and less steps and skill for a caregiver to deploy the dressing assembly 402 at a tissue site. Further, the omission of an additional covering, draping, or sealing step involving the placement of an additional sealing structure over the dressing assembly 402 or the dressing bolster 404 avoids the possibility of a leak being associated with the additional sealing structure, which can occur, for example, through wrinkling, caregiver error, or in applications around complex anatomical geometries.

Further, the pre-formed or contoured configuration of some examples of the dressing bolster 404 can conform to complex anatomical geometries, such as, without limitation, a shoulder, ankle, breast, hand, or sacral region of a patient without causing tissue irritation or blistering. In contrast, a traditional dressing having a generally flat or planar relaxed configuration must be, for example, wrapped, folded, or stretched around complex, multi-dimensional tissue sites, which can generate forces or pulling on tissue at the tissue site in undesirable locations and directions as the traditional dressing naturally attempts to return to its relaxed flat configuration. The dressing assembly 402 can improve or eliminate these problems. For example, the tissue housing 428 on the second side 422 of the dressing bolster 404 can be pre-formed, contoured, or sized to accept a complex or multi-dimensional tissue site, such as the non-limiting shoulder, ankle, breast, hand, or sacral region of a patient described above. The contoured configuration of the dressing bolster 404 therefore prevents pulling and other forces on the tissue from occurring in an undesirable manner, which also improves sealing ability and usability.

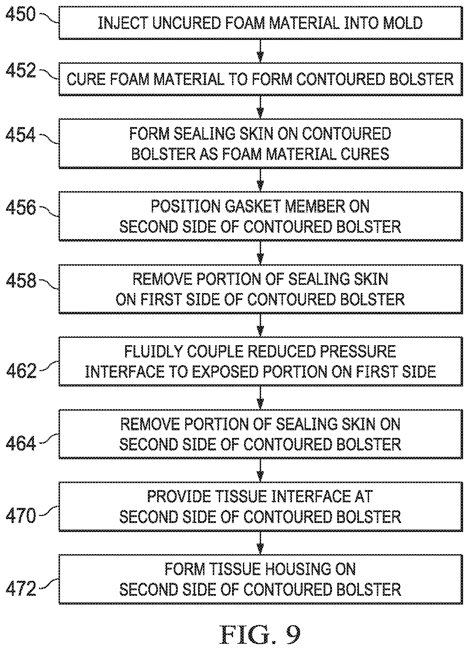

Referring to FIGURE 9, in some example embodiments, a method of manufacturing a dressing assembly 402 according to this disclosure may include a step 450 of injecting an uncured foam material into a mold. Further, the method may include a step 452 of curing the foam material to form the contoured bolster 404 according to a shape of the mold. As described herein, the contoured bolster 404 may include the first side 420, the second side 422 opposite the first side 420, and the edge 424 between the first side 420 and the second side 422. Further, the method may include a step 454 of forming the sealing skin 406 on at least a portion of the first side 420 of the contoured bolster 404 as the foam material cures, and a step 456 of positioning the gasket member 408 proximate to the edge 424 and on the second side 422 of the contoured bolster 404.

In some examples, the method may include a step 458 of removing a first sealing skin portion of the sealing skin 406 on the first side 420 of the contoured bolster 404 to expose a first exposed portion 460 of the contoured bolster 404, shown in FIGURE 7. The removed first sealing skin portion is not shown since it is removed on the first side 420 and discarded to form the first exposed portion 460. The first exposed portion 460 of the contoured bolster 404 may be configured to receive reduced pressure and to distribute the reduced pressure through the contoured bolster 404 to the second side 422 of the contoured bolster 404. Further, the method may include a step 462 of fluidly coupling the reduced pressure interface 138 to the first exposed portion 460 of the contoured bolster 404 on the first side 420 of the contoured bolster 404. The reduced pressure interface 138 may be configured to be coupled in fluid communication with the reduced-pressure source 144 as described herein.

In some examples, the sealing skin 406 may be formed on the first side 420, the second side 422, and the edge 424 of the contoured bolster 404. In such an example, the method may include a step 464 of removing a second sealing skin portion of the sealing skin 406 on the second side 422 of the contoured bolster 404 to expose a second exposed portion 468 of the contoured bolster 404 on the second side 422. The second exposed portion 468 is shown in FIGURE 7 in dash line as being covered by the optional tissue interface 410. The second exposed portion 468 of the contoured bolster 404 may be configured to face the tissue site 102 and to be positioned in fluid communication with the tissue site 102 directly or through the optional tissue interface 410.

Further, in some examples, the method may include a step 470 of providing the tissue interface 410 at the second side 422 of the contoured bolster 404. The tissue interface 410 may be configured to be positioned in contact with the tissue site 102 as described herein. Further, in some examples, the method may include a step 472 of forming the tissue housing 428 on the second side 422 of the contoured bolster 404. Some examples of the tissue housing 428 may include the three-dimensional contact surface 430 configured to receive at least a portion of the tissue site 102 and to contact the tissue site 102 in three-dimensions as described herein. By way of non-limiting example, the shape of the tissue housing 428 may be formed by molding or cutting.

## Claims

1. A system (100) for treating atissue site (102), comprising:
a dressing assembly (112), comprising:
a dressing bolster (114) including a first side (120) and an opposing second side (122), the second side (122) configured to be positioned in fluid communication with the tissue site (102), wherein the dressing bolster (114) has a contoured, non-planar, shape and includes a tissue housing (428) on the second side (122) of the dressing bolster (114), the tissue housing (428) including a three-dimensional contact surface (430) configured to receive at least a portion of the tissue site (102) and to contact the tissue site (102) in three-dimensions,
a sealing skin (116) covering at least a portion of the first side (120) of the dressing bolster (114), wherein the sealing skin (116) is liquid impermeable and integrally formed with the dressing bolster (114), and
a gasket member (117) configured to create a sealed space (342) between the sealing skin (116) and the tissue site (102); and
a reduced-pressure source (118) configured to be coupled in fluid communication with the dressing bolster (114) through the sealing skin (116).

2. The system (100) of claim 1, wherein the second side (122) of the dressing bolster (114) is configured to face the tissue site (102).

3. The system (100) of claim 1, wherein the second side (122) of the dressing bolster (114) is configured to be positioned in contact with the tissue site (102).

4. The system (100) of claim 1, wherein the dressing bolster (114) is configured to be positioned in the sealed space (342) at the tissue site (102).

5. The system (100) of claim 1, wherein the three-dimensional contact surface (430) includes a concave shape.

6. The system (100) of claim 1, wherein the dressing bolster (114) is comprised of an open-cell foam.

7. The system (100) of claim 1, further comprising a tissue interface (410) coupled to the second side (122) of the dressing bolster (114) and configured to be positioned in contact with the tissue site (102).

8. The system (100) of claim 7, wherein the tissue interface (410) is selected from the group consisting of a woven material, a non-woven material, a polyester knit material, a fenestrated film, a felted foam, and a foam skin.

9. The system (100) of claim 1, wherein the sealing skin (116) covers the first side (120) of the dressing bolster (114), an edge of the dressing bolster (114) between the first side (120) and the second side (122) of the dressing bolster (114), and a portion of the second side (122) of the dressing bolster (114).

10. The system (100) of claim 1, wherein the dressing bolster (114) comprises a porous foam, and wherein the sealing skin (116) comprises a liquid impermeable surface formed on and from a portion of the porous foam.

11. The system (100) of claim 1, wherein the gasket member (117) is positioned on a portion of the second side (122) of the dressing bolster (114).

12. The system (100) of claim 1, wherein a portion of the sealing skin (116) is positioned between the second side (122) of the dressing bolster (114) and the gasket member (117).

13. The system (100) of claim 1, wherein the gasket member (117) comprises a hydrocolloid.

14. The system (100) of claim 1, wherein the gasket member (117) is positioned around a circumference of the dressing bolster (114) and configured to surround the tissue site (102).

15. The system (100) of claim 1, wherein the gasket member (117) is positioned at an edge of the dressing bolster (114).

## Patentansprüche

1. Ein System (100) zum Behandeln einer Gewebestelle (102), aufweisend:
eine Verbandanordnung (112), aufweisend:
ein Verbandpolster (114), das eine erste Seite (120) und eine gegenüberliegende zweite Seite (122) aufweist, wobei die zweite Seite (122) konfiguriert ist, um in Fluidverbindung mit der Gewebestelle (102) positioniert zu werden, wobei das Verbandpolster (114) eine konturierte, nicht ebene Form aufweist und ein Gewebegehäuse (428) auf der zweiten Seite (122) des Verbandpolsters (114) aufweist, wobei das Gewebegehäuse (428) eine dreidimensionale Kontaktoberfläche (430) aufweist, die konfiguriert ist, um mindestens einen Abschnitt der Gewebestelle (102) aufzunehmen und die Gewebestelle (102) in drei Dimensionen zu berühren,
eine Dichtungshaut (116), die mindestens einen Abschnitt der ersten Seite (120) des Verbandpolsters (114) abdeckt, wobei die Dichtungshaut (116) flüssigkeitsundurchlässig und einstückig mit dem Verbandpolster (114) ausgebildet ist, und
ein Dichtungselement (117), das konfiguriert ist, um einen abgedichteten Raum (342) zwischen der Dichtungshaut (116) und der Gewebestelle (102) zu erzeugen; und
eine Unterdruckquelle (118), die konfiguriert ist, um in eine Fluidverbindung mit dem Verbandpolster (114) durch die Dichtungshaut (116) gekoppelt zu werden.

2. Das System (100) nach Anspruch 1, wobei die zweite Seite (122) des Verbandpolsters (114) konfiguriert ist, um der Gewebestelle (102) zugewandt zu sein.

3. Das System (100) nach Anspruch 1, wobei die zweite Seite (122) des Verbandpolsters (114) konfiguriert ist, um in Kontakt mit der Gewebestelle (102) positioniert zu werden.

4. Das System (100) nach Anspruch 1, wobei das Verbandpolster (114) konfiguriert ist, um in dem abgedichteten Raum (342) an der Gewebestelle (102) positioniert zu werden.

5. Das System (100) nach Anspruch 1, wobei die dreidimensionale Kontaktoberfläche (430) eine konkave Form aufweist.

6. Das System (100) nach Anspruch 1, wobei das Verbandpolster (114) aus einem offenzelligen Schaum besteht.

7. Das System (100) nach Anspruch 1, ferner aufweisend eine Gewebeschnittstelle (410), die mit der zweiten Seite (122) des Verbandpolsters (114) gekoppelt und konfiguriert ist, um in Kontakt mit der Gewebestelle (102) positioniert zu werden.

8. Das System (100) nach Anspruch 7, wobei die Gewebeschnittstelle (410) aus der Gruppe ausgewählt ist, bestehend aus einem gewebten Material, einem nicht gewebten Material, einem Polyesterstrickmaterial, einer gefensterten Folie, einem gefilzten Schaum und einer Schaumstoffhaut.

9. Das System (100) nach Anspruch 1, wobei die Dichtungshaut (116) die erste Seite (120) des Verbandpolsters (114), einen Rand des Verbandpolsters (114) zwischen der ersten Seite (120) und der zweiten Seite (122) des Verbandpolsters (114) und einen Abschnitt der zweiten Seite (122) des Verbandpolsters (114) abdeckt.

10. Das System (100) nach Anspruch 1, wobei das Verbandpolster (114) einen porösen Schaum aufweist, und wobei die Dichtungshaut (116) eine flüssigkeitsundurchlässige Oberfläche aufweist, die auf und aus einem Abschnitt des porösen Schaums ausgebildet wird.

11. Das System (100) nach Anspruch 1, wobei das Dichtungselement (117) auf einem Abschnitt der zweiten Seite (122) des Verbandpolsters (114) positioniert ist.

12. Das System (100) nach Anspruch 1, wobei ein Abschnitt der Dichtungshaut (116) zwischen der zweiten Seite (122) des Verbandpolsters (114) und dem Dichtungselement (117) positioniert ist.

13. Das System (100) nach Anspruch 1, wobei das Dichtungselement (117) ein Hydrokolloid aufweist.

14. Das System (100) nach Anspruch 1, wobei das Dichtungselement (117) um einen Umfang des Verbandpolsters (114) herum positioniert und konfiguriert ist, um die Gewebestelle (102) zu umgeben.

15. Das System (100) nach Anspruch 1, wobei das Dichtungselement (117) an einem Rand des Verbandpolsters (114) positioniert ist.

## Revendications

1. Système (100) destiné à traiter un site tissulaire (102), comprenant :
un ensemble pansement (112) comprenant :
un support de pansement (114) comportant un premier côté (120) et un second côté opposé (122), le second côté (122) étant conçu pour être positionné en communication fluidique avec le site tissulaire (102), dans lequel le support de pansement (114) a une forme profilée, non plane et comporte un logement de tissu (428) sur le second côté (122) du support de pansement (114), le logement de tissu (428) comportant une surface de contact tridimensionnelle (430) conçue pour recevoir au moins une partie du site tissulaire (102) et pour entrer en contact avec le site tissulaire (102) en trois dimensions,
une peau d'étanchéité (116) recouvrant au moins une partie du premier côté (120) du support de pansement (114), dans lequel la peau d'étanchéité (116) est imperméable aux liquides et formée d'un seul tenant avec le support de pansement (114), et
un élément de joint (117) conçu pour créer un espace scellé (342) entre la peau d'étanchéité (116) et le site tissulaire (102) ; et
une source de pression réduite (118) conçue pour être accouplée en communication fluidique au support de pansement (114) à travers la peau d'étanchéité (116).

2. Système (100) selon la revendication 1, dans lequel le second côté (122) du support de pansement (114) est conçu pour faire face au site tissulaire (102).

3. Système (100) selon la revendication 1, dans lequel le second côté (122) du support de pansement (114) est conçu pour être positionné en contact avec le site tissulaire (102).

4. Système (100) selon la revendication 1, dans lequel le support de pansement (114) est conçu pour être positionné dans l'espace scellé (342) au niveau du site tissulaire (102).

5. Système (100) selon la revendication 1, dans lequel la surface de contact tridimensionnelle (430) comporte une forme concave.

6. Système (100) selon la revendication 1, dans lequel le support de pansement (114) est constitué d'un plastique poreux.

7. Système (100) selon la revendication 1, comprenant en outre une interface tissulaire (410) accouplée au second côté (122) du support de pansement (114) et conçue pour être positionnée en contact avec le site tissulaire (102).

8. Système (100) selon la revendication 7, dans lequel l'interface de tissu (410) est choisie à partir du groupe constitué d'un matériau tissé, d'un matériau non tissé, d'un matériau tricoté de polyester, d'un film fenêtré, d'une mousse feutrée et d'une peau de mousse.

9. Système (100) selon la revendication 1, dans lequel la peau d'étanchéité (116) recouvre le premier côté (120) du support de pansement (114), un bord du support de pansement (114) entre le premier côté (120) et le second côté (122) du support de pansement (114), et une partie du second côté (122) du support de pansement (114).

10. Système (100) selon la revendication 1, dans lequel le support de pansement (114) comprend une mousse poreuse, et dans lequel la peau d'étanchéité (116) comprend une surface imperméable aux liquides formée sur une partie de la mousse poreuse et à partir de celle-ci.

11. Système (100) selon la revendication 1, dans lequel l'élément de joint (117) est positionné sur une partie du second côté (122) du support de pansement (114).

12. Système (100) selon la revendication 1, dans lequel une partie de la peau d'étanchéité (116) est positionnée entre le second côté (122) du support de pansement (114) et l'élément de joint (117).

13. Système (100) selon la revendication 1, dans lequel la couche polymère (117) comprend un hydrocolloïde.

14. Système (100) selon la revendication 1, dans lequel l'élément de joint (117) est positionné autour d'une circonférence du support de pansement (114) et conçu pour entourer le site tissulaire (102).

15. Système (100) selon la revendication 1, dans lequel l'élément de joint (117) est positionné au niveau d'un bord latéral du support de pansement (114).
